# FASCICULE DE BREVET EUROPEEN

(11) **EP 1 688 124 B1**
(45) Date de publication et mention de la délivrance du brevet: **17.10.2007**
(21) Numéro de dépôt: 06290215.0
(22) Date de dépôt: 07.02.2006
(51) Int. Cl.: A61J 1/00, A01N 1/02, A45C 11/00, A45C 13/02

(54) **Sacoche pour le transport de greffons en vue de transplantations**
Behälter für den Transport von Transplantaten
Container for the transport of transplants

(30) Priorité: 08.02.2005 FR 0501253
(43) Date de publication de la demande: 09.08.2006
(73) Titulaire: E3 Cortex, 77230 Thieux (FR); Institut Georges Lopez, 69380 Civrieux d'Azergues (FR)
(72) Inventeur: Pascal, Christian, 95270 Seugy (FR); Samy, Jérôme, 93700 Drancy (FR)
(74) Mandataire: Eidelsberg, Olivier Nathan

(56) Documents cités:
- EP-A- 0 256 730
- WO-A-02/18210
- US-A- 4 502 295
- US-A- 4 951 482
- US-A- 5 441 170

## Description

La présente invention vise une sacoche destinée au transport de greffons pour la transplantation.

Une telle sacoche est connue du document US 4951482.

L'un des buts de l'invention est de réaliser une sacoche qui offre une grande sécurité pour le transport de greffons.

La sacoche suivant l'invention est telle que définie à la revendication 1, des perfectionnements étant définis aux sous revendications.

La sacoche selon un perfectionnement de l'invention peut également être caractérisée en ce qu'elle comporte une paroi interne délimitant deux compartiments, deux parties ouvrables, une pour chaque compartiment, chaque partie ouvrable étant fermable par une fermeture à curseur, le premier compartiment étant destiné à recevoir un flacon du sang du donneur protégé par un bloc de matière souple et élastique et un emballage destiné à contenir un pot pour recevoir des glandes du donneur, tandis que le second compartiment contient une cuve isotherme fermée par un couvercle isotherme et destinée à recevoir un pot stérile contenant le greffon et logé dans un emballage stérile, ladite sacoche comportant, sur une face latérale adjacente au premier compartiment, une pochette transparente pour contenir un dossier sur le donneur, tandis que la partie ouvrante du second compartiment, sur sa face extérieure, est pourvue d'une pochette transparente destinée à recevoir une étiquette normalisée et, sur la face intérieure, une autre pochette transparente pour recevoir le dossier du donneur.

De préférence, les pochettes sont fermées par des rabats avec des fermetures du type "Velcro".

La sacoche peut être pourvue d'une bandoulière.

Le second compartiment comporte des sangles pour le maintien du couvercle de la cuve isotherme.

Suivant une autre caractéristique, les pots comportent un couvercle vissé pourvu d'une anse, tandis que le fond est pourvu de crans sur sa surface externe.

Enfin, le curseur de chaque fermeture comporte un anneau permettant de la sceller avec la fermeture voisine. Ainsi, on est assuré que, durant le transport, les différents compartiments n'ont pas été ouverts.

L'invention va maintenant être décrite avec plus de détails en se référant à un mode de réalisation particulier donné à titre d'exemple seulement et représenté aux dessins annexés.

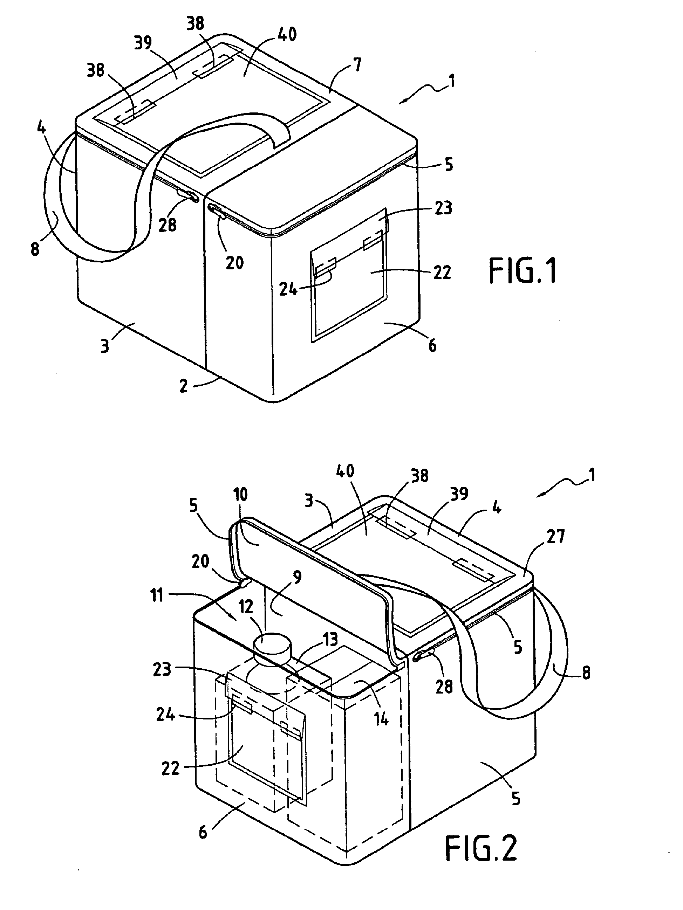

Figure 1 st une vue en perspective d'une sacoche, selon l'invention.
Figure 2 montre la sacoche en perspective avec un premier compartiment ouvert.
Figure 3 est une vue en perspective de l'un des contenants du premier compartiment.
Figure 4 est une vue en perspective éclatée d'un second contenant du premier compartiment.
Figure 5 montre en perspective la sacoche avec le second compartiment ouvert.
Figure 6 est une vue éclatée des éléments contenus dans le second compartiment.
Figure 7 est une vue en perspective d'un détail.

La sacoche représentée aux figures et référencée en 1 est sensiblement parallélépipédique et comprend un fond 2, des parois latérales 3, 4, 5 et 6 et une paroi supérieure 7.

Sur la paroi 4 est fixée une bandoulière 8 fixée sous le fond 2 sur une paroi interne 9 et ressortant par la paroi supérieure 7.

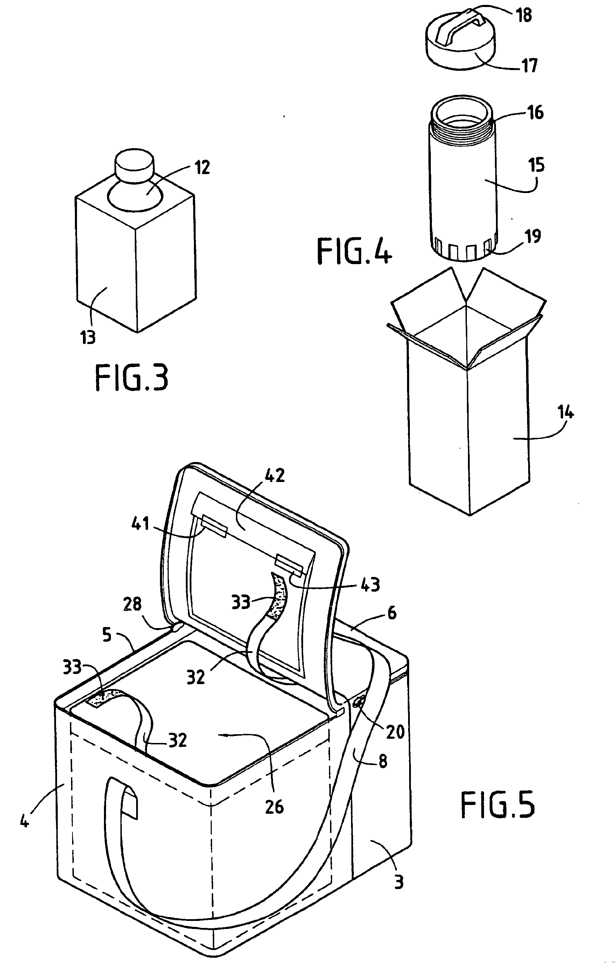

La sacoche est séparée en deux compartiments par la paroi interne 9, le premier compartiment 11 présente une partie ouvrable 10 et est destiné à recevoir un flacon 12 destiné à contenir un tube de sang du donneur afin de pouvoir contrôler la compatibilité donneur receveur ; le flacon 12 est logé dans un bloc 13 de matière souple et élastique (figure 3).

Dans le compartiment 11 est logé un emballage en carton 14 destiné à recevoir un ou plusieurs pots 15.

Le pot 15 est pourvu, à une extrémité, d'un filetage 16 destiné au vissage d'un couvercle 17 pourvu d'une anse 18, tandis que l'autre extrémité présente des crans 19. Ainsi, pour visser ou dévisser le couvercle, le corps du flacon 15 peut être tenu d'une main dont les doigts coopèrent avec les crans, tandis que l'autre main tient l'anse 18.

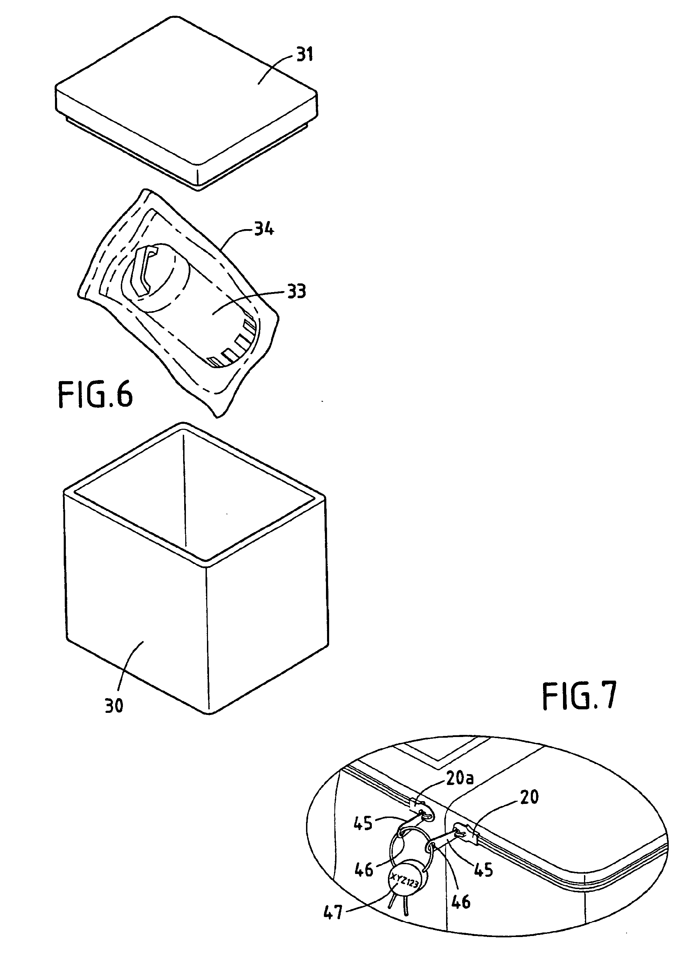

Sur la figure 4, on a représenté un seul flacon, mais l'emballage 14 pourrait recevoir, par exemple, deux flacons plus petits destinés à recevoir, l'un la rate du donneur et l'autre les ganglions.

La partie ouvrable 10 du compartiment 11 est fermée par une fermeture à curseur 20.

Le côté 6 est pourvu d'une poche transparente 22 fermée par un rabat 23 avec des fermetures dites "Velcro" 24 et destinée à recevoir un dossier sur le donneur.

La paroi interne 9 délimite un second compartiment 26, la paroi supérieure 8 présentant une ouverture délimitant une partie ouvrable 27 qui est fermable par une fermeture à curseur 28.

Ce compartiment 26 contient une cuve isotherme 30 fermée par un couvercle isotherme 31 permettant une tenue en température comprise entre 2 et 4°C pendant une durée de 48 heures à 72 heures.

Le couvercle est maintenu par des sangles 32 prévues dans le compartiment 26 et les extrémités sont pourvues de fermetures du type "Velcro" 33.

La cuve isotherme 30 est destinée à recevoir un pot stérile 33 logé dans un emballage stérile 34.

Le pot 33 est du même type que le pot 15 et est destiné à recevoir un organe à greffer.

La face supérieure de la partie ouvrable 27 comporte une pochette transparente 40 fermée par un rabat 39 avec des fermetures du type "Velcro" 38.

Cette pochette est destinée à recevoir une étiquette normalisée afin de pouvoir consulter les indications utiles sans aucune manipulation de la sacoche.

Il est également prévu une pochette transparente 41 sur la face interne de la partie ouvrable 27 avec un rabat 42 et des fermetures du type "Velcro" 43, cette pochette étant destinée à recevoir le dossier du donneur.

La sacoche est réalisée en un tissu polyéthylène doublé.

Pour chaque fermeture à curseur 20, 28 (voir figure 7), les curseurs 20a et 28a comportent des pattes 45 pourvues d'anneaux 46 de manière que les pattes 45 puissent être reliées ensemble par un moyen 47 permettant de les sceller et ainsi on est assuré que, durant le transport, la sacoche n'a pas été ouverte.

Bien entendu, l'invention n'est pas limitée au mode de réalisation qui vient d'être décrit et représenté. On pourra y apporter de nombreuses modifications de détail sans sortir pour cela du cadre de l'invention.

## Revendications

1. Sacoche pour le transport de greffons en vue de transplantations **caractérisée en ce qu**'elle comporte au moins une paroi interne (9) délimitant au moins deux compartiments (11, 26) ayant chacun une partie ouvrable (10, 27), un premier compartiment (11) étant destiné à recevoir un flacon du sang du donneur protégé par un bloc de matière souple et élastique (13) et un emballage (14) destiné à contenir un pot (15) pour recevoir des glandes du donneur, tandis qu'un deuxième compartiment contient une cuve isotherme (30) destinée à recevoir un pot stérile (33) contenant le greffon.

2. Sacoche suivant la revendication 1, **caractérisée en ce qu**'elle comporte sur une face latérale, de préférence adjacente au premier compartiment (11), une pochette transparente (22) pour contenir un dossier sur le donneur, tandis que la partie ouvrante du deuxième compartiment (26), de préférence sur sa face extérieure, est pourvue d'une première pochette (40), de préférence transparente, destinée à recevoir une étiquette normalisée et, de préférence sur sa face intérieure, une deuxième pochette (41), de préférence transparente, pour recevoir le dossier du donneur.

3. Sacoche suivant la revendication 1 ou 2, **caractérisée en ce que** les parties ouvrables sont fermées par une fermeture à curseur.

4. Sacoche pour le transport des greffons en vue de transplantations, selon l'une des revendications 1 à 3, **caractérisée en ce que** les pochettes sont fermées par des rabats avec des fermetures du type "Velcro".

5. Sacoche pour le transport des greffons en vue de transplantations selon l'une des revendications 1 à 4, **caractérisée en ce que** la sacoche est pourvue d'une bandoulière (8).

6. Sacoche pour le transport des greffons en vue de transplantations selon l'une des revendications 1 à 5, **caractérisée en ce que** le deuxième compartiment (26) comporte des sangles pour le maintien d'un couvercle (31) de la cuve isotherme (30).

7. Sacoche pour le transport des greffons en vue de transplantations selon l'une des revendications 1 à 6, **caractérisée en ce que** les pots (15, 33) logés dans les compartiments comportent un couvercle (17) vissé pourvu d'une anse (18), tandis que le fond est pourvu de crans sur sa surface externe.

8. Sacoche pour le transport des greffons en vue de transplantations selon l'une des revendications 3 à 7, **caractérisée en ce que** chaque fermeture à curseur comporte un anneau (46) permettant de la sceller avec la fermeture voisine.

## Claims

1. Container for transporting transplant grafts, **characterised in that** it comprises at least an internal wall (9) delimiting at least two compartments (11, 26) each having an opening part (10, 27), a first compartment (11) being intended to receive a bottle of the donor's blood protected by a flexible resilient block (13) and packaging (14) intended to receive a jar (15) for receiving the donor's glands, whilst a second compartment contains an isothermal tank (30) for receiving a sterile jar (33) containing the graft.

2. Container according to claim 1, **characterised in that** it comprises, on a lateral face preferably adjacent to the first compartment (11), a transparent pocket (22) for containing a donor's file, whilst the opening part of the second compartment (26), preferably on its external face, is provided with a first, preferably transparent, pocket (40) for receiving a standard label and, preferably on its internal face, a second, preferably transparent, pocket (41) for receiving the donor's file.

3. Container according to either claim 1 or claim 2, **characterised in that** the opening parts are closed by means of a zip fastener.

4. Container for transporting transplant grafts according to any one of claims 1 to 3, **characterised in that** the pockets are closed by means of flaps with Velcro-type fasteners.

5. Container for transporting transplant grafts according to any one of claims 1 to 4, **characterised in that** the container is provided with a shoulder strap (8).

6. Container for transporting transplant grafts according to any one of claims 1 to 5, **characterised in that** the second compartment (26) comprises straps for holding in place a lid (31) of the isothermal tank (30).

7. Container for transporting transplant grafts according to any one of claims 1 to 6, **characterised in that** the jars (15, 33) placed in the compartments comprise a screw-on lid (17) provided with a handle (18), whilst the bottom is provided with notches on its external surface.

8. Container for transporting transplant grafts according to any one of claims 3 to 7, **characterised in that** each zip fastener comprises a ring (46) enabling it to be locked to the neighbouring zip fastener.

## Patentansprüche

1. Behälter für den Transport von Transplantaten im Hinblick auf Transplantationen, **dadurch gekennzeichnet, dass** er mindestens eine innere Wand (9) aufweist, die mindestens zwei Fächer (11, 26) begrenzt, wobei jedes ein öffenbares Teilstück (10, 27) aufweist, wobei ein erstes Fach (11) für die Aufnahme einer durch einen anpassungsfähigen und elastischen Block (13) geschützten Flasche Blut des Spenders und einer Verpackung (14) für eine Kanne (15) zur Aufnahme von Drüsen des Spenders bestimmt ist, während ein zweites Fach einen Thermosbehälter (30) enthält, der zur Aufnahme einer sterilen Kanne (33), welche das Transplantat enthält, bestimmt ist.

2. Behälter nach Anspruch 1, **dadurch gekennzeichnet, dass** er auf einer Seitenwand, bevorzugt angrenzend an das erste Fach (11), eine Klarsichttasche (22) aufweist zur Aufnahme einer Akte zum Spender, während das öffenbare Teilstück des zweiten Fachs (26), bevorzugt auf seiner Außenseite, mit einer ersten Tasche (40) ausgestattet ist, bevorzugt klarsichtig, bestimmt zur Aufnahme eines genormten Etiketts und bevorzugt auf seiner Innenseite eine zweite Tasche (41), bevorzugt klarsichtig, zur Aufnahme der Akte des Spenders.

3. Behälter nach den Ansprüchen 1 oder 2, **dadurch gekennzeichnet, dass** die öffenbaren Teilstücke durch einen Schiebeverschluss geschlossen sind.

4. Behälter für den Transport von Transplantaten im Hinblick auf Transplantationen nach einem der Ansprüche 1 bis 3, **dadurch gekennzeichnet, dass** die Taschen durch Klappen mit Verschlüssen vom Typ "Velcro" geschlossen sind.

5. Behälter für den Transport von Transplantaten im Hinblick auf Transplantationen nach einem der Ansprüche 1 bis 4, **dadurch gekennzeichnet, dass** der Behälter mit einem Trageband (8) ausgestattet ist.

6. Behälter für den Transport von Transplantaten im Hinblick auf Transplantationen nach einem der Ansprüche 1 bis 5, **dadurch gekennzeichnet, dass** das zweite Fach (26) Gurte aufweist zum Festhalten eines Deckels (31) des Thermosbehälters (30).

7. Behälter für den Transport von Transplantaten im Hinblick auf Transplantationen nach einem der Ansprüche 1 bis 6, **dadurch gekennzeichnet, dass** die Kannen (15, 33) die in den Fächern lagern, einen geschraubten Deckel (17) aufweisen, der mit einem Henkel (18) versehen ist, während der Boden auf seiner Oberfläche mit Einkerbungen versehen ist.

8. Behälter für den Transport von Transplantaten im Hinblick auf Transplantationen nach einem der Ansprüche 3 bis 7, **dadurch gekennzeichnet, dass** jeder Schiebeverschluss eine Öse (46) aufweist, die es erlaubt, ihn mit dem benachbarten Verschluss zu versiegeln.
